# EUROPÄISCHE PATENTSCHRIFT

(11) **EP 0 940 145 B1**
(45) Veröffentlichungstag und Bekanntmachung des Hinweises auf die Patenterteilung: **09.04.2003**
(21) Anmeldenummer: 99103770.6
(22) Anmeldetag: 26.02.1999
(51) Int. Cl.: A61L 2/00, A61L 2/16, A61L 2/18, A61L 2/22

(54) **O/W-Mikroemulsionen mit einem Gehalt an einem oder mehreren Alkylthiouronium und/oder alpha-omega-Alkylendithiouroniumsalzen als mikrobiozide Wirkstoffe sowie die Verwendung derselben zur manuellen und/oder maschinellen Instrumentendesinfektion**
O/W microemulsions containing one or more salts of alkylthiouronium and/or alpha-omega-alkylendithiouronium as microbiocidal agents and their use for manual or automated disinfection of instruments
Microémulsions huile-dans-l'eau contenant un ou plusieurs sels d' alkylthiouronium et/ou d'alpha-omega-alkylendithiouronium comme agents microbicides et leur utilisation pour la désinfection des instruments manuelle et / ou par machine

(30) Priorität: 04.03.1998 DE 19808964
(43) Veröffentlichungstag der Anmeldung: 08.09.1999
(73) Patentinhaber: Bode Chemie GmbH & Co., 22525 Hamburg (DE)
(72) Erfinder: Jülich, Wolf-Dieter Dr., 17489 Greifswald (DE); Seibt, Horst Dr., 10407 Berlin (DE); Pietsch, Hanns Dr., 20148 Hamburg (DE); Kramer, Exel Prof., 17487 Greifswald (DE); Ohme, Roland Dr., 12527 Berlin (DE); Ballschuh, Detlef Dr., 12524 Berlin (DE); Knieler, Roland Dr., 22529 Hamburg (DE)
(74) Vertreter: Dinné, Erlend

(56) Entgegenhaltungen:
- EP-A- 0 842 606
- GB-A- 768 067
- GB-A- 815 827
- GB-A- 1 018 304
- GB-A- 1 551 224
- US-A- 5 674 561

## Beschreibung

Die vorliegende Erfindung betrifft O/W-Mikroemulsionen mit einem Gehalt an einem oder mehreren Alkylthiouronium- und/oder α, ω-Alkylendithiouroniumsalzen, ihre Verwendung als mikrobizide Wirkstoffe sowie zur manuellen und/oder maschinellen Instrumentendesinfektion.

Als Desinfektionsmittel zur Desinfektion von Instrumenten und Wäsche sind eine Vielzahl mikrobizid wirksamer chemischer Substanzen bzw. Gemische dieser Substanzen an sich bekannt. Mikrobizide Substanzen sind im allgemeinen gegen das übliche Spektrum von Keimen, wie beispielsweise grampositive Bakterien, gramnegative Bakterien, Mykobakterien, Hefen, Pilze, Viren und dergleichen, mehr oder weniger wirksam, so daß man üblicherweise eine ausreichende Desinfektion durch geeignete Wirkstoffkombinationen erzielen kann.

Der Stand der Technik kennt zur Desinfektion von medizinisch-chirurgischen Instrumenten eine Reihe von Wirkstoffen, insbesondere Aldehyde, wie beispielsweise Formaldehyd oder Glutaraldehyd, quaternäre Ammoniumverbindungen und langkettige Amine sowie Phenole. Allerdings weisen die genannten Verbindungen einige Nachteile auf:

Aldehyde fixieren Reste von Blut und Eiweiß durch chemische Reaktion an den zu desinfizierenden Gegenständen, so daß diese nach der Desinfektion schwer zu reinigen sind. Außerdem haben Aldehyde ein vergleichsweise hohes allergenes Potential, so daß nur Anwendungen in Kombination mit weiteren Wirkstoffen in Betracht kommen, um die erforderliche Unterschreitung der Sensibilisierungsschwelle einhalten zu können. Die Anwendungsbereiche von Aldehyden werden ferner durch die umstrittene karzinogene Potenz sowie die Mutagenität dieser Stoffklasse weiter eingeschränkt. Zudem haben Aldehyde einen durchdringenden, unangenehmen Geruch und können Schleimhautreizungen bewirken.

Quaternäre Ammoniumverbindungen und langkettige Amine werden häufig in der manuellen Instrumentendesinfektion verwendet. Im Vergleich zu den Aldehyden ist der Geruch dieser Verbindungen deutlich weniger unangenehm. Eine chemische Reaktion mit Eiweißen erfolgt nicht, jedoch kommt es zu einer physikalischen Fällung von Eiweißen, die zum Teil durch geschickte Kombination mit Tensiden kompensiert werden kann. Für die maschinelle Instrumentendesinfektion sind die quaternären Ammoniumverbindungen allerdings nicht geeignet, weil es infolge der Turbulenzen in der Reinigungsmaschine zu einer starken, unerwünschten Schaumbildung kommt. Ein weiterer entscheidender Nachteil ist das eingeengte Wirkungsspektrum quaternärer Ammoniumverbindungen, da diese weder sporozid noch gegen unbehüllte Viren wirken.

Phenole sind vor allem wegen ihres Geruches, ihrer geringen Wirksamkeit gegen den Potiovirus. ihrer zum Teil schlechten Abbaubarkeit, ihrer hohen Lipidlöslichkeit verbunden mit einer starken Penetration durch die Haut sowie toxischer und mutagener Risiken in nahezu allen Anwendungsbereichen für Desinfektionsmittel auf dem Rückzug.

Aufgabe der Erfindung ist es, einen Wirkstoff bzw. eine Wirkstoffkombination zu finden, die eine hohe mikrobizide und sporozide Wirkung zeigt und dabei gleichzeitig die Nachteile des Standes der Technik nicht aufweist, d.h. die insbesondere eine gute Reinigungsleistung bei zugleich geringer Toxizität zeigt, die weder Eiweiß noch Blut an den zu desinfizierenden Gegenständen fixiert, die keinen unangenehmen Geruch hat und in Wasch- und Desinfektionsautomaten für chirurgische Instrumente keine störende Schaumbildung aufweist.

Die hohe antimikrobielle Wirksamkeit von Dodecylthiouroniumchlorid ist an sich bekannt (*Zbl. Bakt. Hyg., 1 Orig. A 237, 117-123, 1977*). Allerdings ist diese Verbindung - ebenso wie andere Thiouroniumsalze - in wäßriger Lösung labil, neigt z.B. in Gegenwart von Metallen zum Ausflocken, und ist daher nur schwer in desinfizierende Formulierungen einzuarbeiten. Für die Instrumentendesinfektion (sowohl für die manuelle als auch die maschinelle Methode) sind Thiouroniumsalze daher nur bedingt geeignet.

Als Thiouroniumsalze werden im folgenden Alkylthiouronium- und/oder α, ω-Alkylendithiouroniumsalze bezeichnet. Alkylthiouroniumsalze zeichnen sich durch folgende chemische Struktur aus 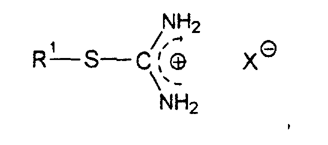 wobei R¹ gewählt wird aus der Gruppe der verzweigten und unverzweigten Alkylgruppen mit 1 bis 20 Kohlenstoffatomen und X⁻ ein beliebiges Anion darstellt, vorzugsweise aber ein Halogenid-Ion.

α, ω-Alkylendithiouroniumsalze sind durch die folgende Struktur gekennzeichnet 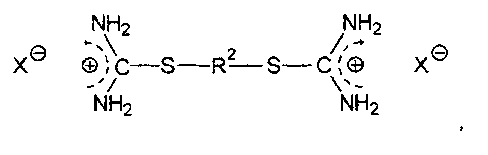 wobei R² gewählt wird aus der Gruppe der verzweigten und unverzweigten Alkylgruppen mit 1 bis 20 Kohlenstoffatomen und X⁻ ein beliebiges Anion darstellt, vorzugsweise aber ein Halogenid-lon.

Aufgrund ihres aliphatischen Molekülteils weisen Thiouroniumsalze einen ausgeprägten Tensidcharakter auf, der eine hohe Reinigungswirkung erwarten läßt. Damit verbunden ist allerdings auch eine starke Schaumbildung, so daß Thiouroniumsalzlösungen für die maschinelle Instrumentendesinfektion bisher nicht eingesetzt werden konnten.

Supramolekulare Strukturen, beispielsweise in Form von Mikroemulsionen, sind an sich bekannt.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca. 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulichweißgefärbt und undurchsichtig.

Der Tröpfchendurchmesser von Mikroemulsionen dagegen liegt im Bereich von etwa 10 nm bis etwa 100 nm. Sie erscheinen, da der Partikeldurchmesser kleiner als ca. 100 nm ist, klar und transparent. Mikroemulsionen sind meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe feiner dispers vorliegen können als in der dispersen Phase von "Makroemulsionen". Ein weiterer Vorteil ist, daß sie aufgrund ihrer niedrigen Viskosität versprühbar sind.

Es war indes überraschend und für den Fachmann nicht vorauszusehen, daß O/W-Mikroemulsionen, gekennzeichnet durch einen Gehalt an mindestens einem Alkylthiouronium- und/oder α,ω-Alkylendithiouroniumsalz den geschilderten Nachteilen des Standes der Technik Abhilfe schaffen würden.

O/W-Mikroemulsionen im Sinne der vorliegenden Erfindung sind besonders geeignet zum Desinfizieren von Instrumenten, insbesondere zur maschinellen Instrumentenaufbereitung. Sie zeichnen sich überraschenderweise insbesondere dadurch aus, daß ihre Verwendung nicht zu dem bekannten Phänomen des unerwünschten Fixierens bzw. Ausfällens von Eiweiß auf den zu desinfizierenden Gegenständen führt.

Dies war insbesondere deswegen nicht zu erwarten, weil Thiouroniumsalze eine hohe strukturelle Ähnlichkeit mit quaternären Ammoniumverbindungen haben, welche bekanntermaßen eine physikalische Eiweißfällung hervorrufen.

Die bakterizide und fungizide Wirkung der erfindungsgemäßen Formulierungen ist ausgesprochen gut. Überraschenderweise ist zusätzlich eine sporozide Wirkung gegeben, obwohl die einzelnen Komponenten nicht sporozid wirksam sind. Von Vorteil für ihre Anwendung im Bereich der maschinellen Instrumentenaufbereitung ist insbesondere ihre inaktivierende Wirkung auf das Hepatitis B-Virus. Das Hepatitis B-Oberflächenantigen wird bereits nach einer Einwirkungszeit von 5 Minuten zerstört.

Überraschenderweise bilden die erfindungsgemäßen Formulierungen im Gegensatz zum Einzelwirkstoff selbst bei starker Turbulenz nur eine sehr geringe Schaummenge und verursachen in ihrer Umgebung keine bzw. nur eine geringe Korrosion. Dies ist naturgemäß insbesondere für die Instrumentendesinfektion von Vorteil, da sowohl die zu desinfizierenden Gegenstände als auch die verwendeten Wasch- und Desinfektionsautomaten im allgemeinen aus Metall gefertigt sind bzw. Metallteile enthalten.

Es ist erfindungsgemäß möglich, die Einsatzmengen an Alkylthiouronium- und/oder α, ω-Alkylendithiouroniumsalzen gegenüber dem Stande der Technik erheblich zu steigern. Vorteilhaft ist es, den Gehalt an einem oder mehreren Alkylthiouronium- und/oder α, ω-Alkylendithiouroniumsalzen in der Mikroemulsion zwischen 0,05 und 30 Gew.-%, besonders vorteilhaft zwischen 5 und 20 Gew.-%, bezogen auf das Gesamtgewicht der Mikroemulsion, zu wählen.

Die erfindungsgemäßen O/W-Mikroemulsionen werden entweder als solche, d. h. in Form von "Konzentraten", oder nach Verdünnen mit Wasser - in Form einer sogenannten "Gebrauchslösung" - als Desinfektionsmittel verwendet. Vorteilhaft ist der Gehalt an einem oder mehreren Alkylthiouronium- und/oder α, ω-Alkylendithiouroniumsalzen in der Gebrauchslösung, die beispielsweise durch Verdünnen mit Wasser erzeugt wird, zwischen 0,01 und 30 Gew.-%, bezogen auf das Gesamtgewicht der Gebrauchslösung, zu wählen.

Die als Konzentrat vorliegenden erfindungsgemäßen O/W-Mikroemulsionen sind vorzugsweise nahezu monodispers mit einer Tröpfchengröße von bevorzugt weniger als 20 nm, insbesondere weniger als 10 nm.

Vorteilhaft im Sinne der vorliegenden Erfindung ist es, die als Konzentrat vorliegenden erfindungsgemäßen O/W-Mikroemulsionen - je nach Wirkstoffgehalt - mit Wasser im Verhältnis von beispielsweise 1 : 50 bis 1 : 200 zu einer Gebrauchslösung zu verdünnen.

Bevorzugt sind O/W-Mikroemulsionen, die neben einem oder mehreren erfindungsgemäßen Wirkstoffen weitere antimikrobielle Wirkstoffe enthalten. Alkylthiouronium- und/oder α,ω-Alkylendithiouroniumsalze sind geeignete Kombinationspartner für nahezu alle bekannten Wirkstoffe. (Eine Ausnahme bilden Aldehyde, die mit den NH₂-Gruppen reagieren.) Ein besonders vorteilhafter Kombinationspartner im Sinne der vorliegenden Erfindung ist Salicylsäure.

Die erfindungsgemäßen O/W-Mikroemulsionen enthalten einen oder mehrere Emulgatoren.

Vorteilhaft werden der oder die Emulgatoren aus der Gruppe der nichtionischen Emulgatoren, insbesondere der oxethylierten geradkettigen Alkohole (der sogenannten Fettalkylpolyoxyalkylene) wie z. B. Fettsäureethoxylat, Fettsäureethoxylat/propoxylat oder Fettsäureethoxylat/propoxylat/butoxylat, gewählt. Vorteilhaft sind beispielsweise die unter der Handelsbezeichnung Plurafac® LF 223 bei der BASF AG erhältlichen Fettalkylpolyoxybutylenpolyoxyethylene. Bevorzugt im Sinne der vorliegenden Erfindung sind ferner Fettalkylpolyoxyethylene, beispielsweise solche mit 2 bis 6 EO-Einheiten, wie z. B. Dodecyldioxyethylen.

Es wird bevorzugt, den Gesamtgehalt an Emulgatoren zwischen 0,5 und 2,0 Gew.-%. bezogen auf das Gesamtgewicht der erfindungsgemäßen O/W-Mikroemulsion, zu halten.

Alkylthiouronium- und α, ω-Alkylendithiouroniumsalze neigen als kationische Tenside an sich zu starker Schaumbildung, weshalb es vorteilhaft sein kann, den erfindungsgemäßen Zubereitungen oberflächenaktive Substanzen zuzusetzen, welche im gesamten Temperaturbereich der Anwendung (d. h. zwischen 20 und 60 °C) eine geringe Schaumneigung bzw sogar eine schaumdämpfende Wirkung zeigen. Als solche zusätzlichen Substanzen mit geringer Schaumneigung sind im Sinne der vorliegenden Erfindung beispielsweise die bereits genannten Fettalkylpolyoxyalkylene anzusehen.

Vorteilhafte zusätzliche Substanzen mit sehr geringer Schaumneigung bzw. schaumdämpfender Wirkung sind im Sinne der vorliegenden Erfindung ferner sogenannte "endgruppenverschlossene" Fettalkylpolyoxyalkylene, d. h. solche oxethylierten Alkohole, deren endständige OH-Gruppe verethert oder verestert ist, wodurch im allgemeinen die Hydrophilie der Substanz herabgesetzt wird. Vorteilhafte "endgruppenverschlossene" Fettalkylpolyoxyalkylene sind beispielsweise unter der Handelsbezeichnung Plurafac® LF 131, Plurafac® LF 132, Plurafac® LF 231 und Plurafac® LF 431 bei der BASF AG erhältlich.

Die Ölphase (oder besser: organische - d. h. nicht in Wasser lösliche - Phase) der erfindungsgemäßen O/W-Mikroemulsionen wird vorteilhaft gewählt aus der Gruppe der Ester aus gesättigten und/oder ungesättigten, verzweigten und/oder unverzweigten Alkancarbonsäuren einer Kettenlänge von 3 bis 30 Kohlenstoffatomen und gesättigten und/oder ungesättigten, verzweigten und/oder unverzweigten Alkoholen einer Kettenlänge von 3 bis 30 Kohlenstoffatomen, aus der Gruppe der Ester aus aromatischen Carbonsäuren und gesättigten und/oder ungesättigten, verzweigten und/oder unverzweigten Alkoholen einer Kettenlänge von 3 bis 30 Kohlenstoffatomen. Solche Esteröle können vorteilhaft gewählt werden aus der Gruppe Isopropylmyristat, Isopropylpalmitat, Isopropylstearat. Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester.

Es ist erfindungsgemäß vorteilhaft, den Ölphasenanteil der erfindungsgemäßen Mikroemulsionen aus dem Bereich von 5 bis 40 Gew.-%, insbesondere von 10 bis 30 Gew.-% zu wählen, bezogen auf das Gesamtgewicht der Mikroemulsionen.

Es kann auch von Vorteil sein, in erfindungsgemäßen Zubereitungen Lösungsvermittler einzusetzen, beispielsweise solche, die üblicherweise in Desinfektionsmitteln enthalten sind. Vorteilhafte Lösungsvermittler sind beispielsweise aliphatisch-aromatische Alkohole, wie z.B. Phenoxyethanol, Phenoxypropanol und/oder Benzylalkohole, sowie aliphatische Alkohole, wie beispielsweise n-Propanol, iso-Propanol, Butanol-2, Butanol-1 und/oder iso-Butanol. Es wird bevorzugt, den Gehalt an einem oder mehreren Lösungsvermittlern zwischen 3 und 10 Gew.-%, insbesondere zwischen 5 und 10 Gew.-%, bezogen auf das Gesamtgewicht der Mikroemulsion, zu wählen.

Günstig sind auch solche O/W-Mikroemulsionen, die neben Wasser- und Ölen weitere Lösungsmittel enthalten, beispielsweise Fettalkohole, wie z.B. Decanol, Dodecanol, Tetradecanol und/oder Hexadecanol, und/oder Hydroxycarbonsäureethylester, wie z.B. Ethyllactat und/oder Ethylcitrat. Es wird bevorzugt, den Gesamtgehalt an weiteren Lösungsmitteln zwischen 0,5 und 8,0 Gew.-%, insbesondere zwischen 2 und 5 Gew.-%. bezogen auf das Gesamtgewicht der Mikroemulsion, zu halten.

Die erfindungsgemäßen O/W-Mikroemulsionen können vorzugsweise auch weitere Tenside enthalten, wie sie üblicherweise in Desinfektionsmitteln Verwendung finden, beispielsweise tensidische Sulfobetaine.Der Gehalt an zusätzlichen Tensiden wird vorteilhaft zwischen 0,1 und 0,6 Gew.-% gewählt, bezogen auf das Gesamtgewicht der Mikroemulsion.

Ferner können die erfindungsgemäßen O/W-Mikroemulsionen vorteilhaft zusätzlich ubliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können. Es wird bevorzugt, den Gehalt an einem oder mehreren Korrosionsinhibitoren zwischen 0,1 und 0,5 Gew.-%, bezogen auf das Gesamtgewicht der Mikroemulsion, zu wählen.

Zusätzlich zu den vorstehend genannten Komponenten können die erfindungsgemäßen O/W-Mikroemulsionen für derartige Zubereitungen übliche Konservierungsstoffe, Farbstoffe, Duftstoffe und/oder andere übliche Hilfsstoffe enthalten. Diese tragen in der Mehrzahl der Fälle nicht zur desinfizierenden Wirkung bei, sondern dienen der Lagerbarkeit sowie ästhetischen Zwecken. Es ist jedoch auch möglich, solche Komponenten zu verwenden, die eine (konservierende, pflegende usw.) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die folgenden Beispiele dienen dazu, die Erfindungen zu beschreiben, selbstverständlich ohne daß beabsichtigt ist, die Erfindungen auf diese Beispiele zu beschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1-4: Für die maschinelle Instrumentendesinfektion geeignete Formulierungen

Untersucht wurden Formulierungen der Zusammensetzung ME 1, ME 2, ME 3 und ME 4 auf bakteriostatische Wirkung:

| | ME 1 | ME 2 | ME 3 | ME 4 |
|---|---|---|---|---|
| 10 Gew.-% Alkylthiouroniumbromid | Alkyl = Decyl | Alkyl = Dodecyl | Alkyl = Decyl | Alkyl = Dodecyl |
| Phenoxyethanol | 5 Gew.-% | 5 Gew.-% | 5 Gew-% | 5 Gew.-% |
| niederer Alkohol | 7,5 Gew.-% Propanol-2 | 7,5 Gew.-% Propanol-2 | 7.5 Gew-% Propanol-1 | 7,5 Gew.-% Propanol-1 |
| Butandiol-2 | 3 Gew.-% | 3 Gew.-% | 3 Gew.-% | 3 Gew.-% |
| Tensid⁽¹⁾ | 4 Gew.-% | 4 Gew.-% | 4 Gew.-% | 4 Gew.-% |
| Tensid⁽²⁾ | 1 Gew.-% | 1 Gew.-% | 1 Gew.-% | 1 Gew.-% |
| Benzotriazol | 0,3 Gew.-% | 0,3 Gew.-% | 0,3 Gew.-% | 0,3 Gew.-% |
| 2-Butyldodecanol-2 | 0,3 Gew.-% | 0,3 Gew.-% | 0,3 Gew.-% | 0,3 Gew.-% |
| Wasser | ad 100 | ad 100 | ad 100 | ad 100 |

| | | | | |
|---|---|---|---|---|
| ⁽¹⁾ Fettalkylpolyoxybutylenpolyoxyethylen mit einem Trübungspunkt von 41 °C | | | | |
| ⁽²⁾ oxethylierter Fettalkohol mit 3 EO-Einheiten | | | | |

Methodik des Agarinkorporationstestes zur Ermittlung der bakteriostatischen Wirksamkeit: Die zu untersuchenden Substanzen wurden in Wasser standardisierter Härte gelöst, gegebenenfalls unter Erwärmen im Wasserbad auf 45 bzw. 75 °C. Die Konzentration der Testsubstanzen im Nähragar wurde in einer geometrischen Reihe auf der Basis 2 abgestuft. Zum Beimpfen der Testplatten wurden die Bakterienstämme 6 h in Caseinpepton-Sojamehlpepton-Lösung bebrütet und so in eine logarithmische Wachstumsphase gebracht. Danach wurden die Agarplatten sternförmig sektorenweise mit jeweils einer "abgestrichenen Öse" beimpft. Der Sektor von Proteus mirabilis wurde durch Gräben abgetrennt, um das Schwärmen zu verhindern. Nach 24 h Bebrütung bei 36 °C erfolgte die Bewertung des Bakterienwachstums unter Vergleich mit substanzfreien Kontrollplatten nach folgendem Schema:
- ++++: ungehemmtes Wachstum
- +++: geringe Wachstumshemmung (maximal 3/4 des Wachstums der Kontrollen)
- ++: mäßige Wachstumshemmung (maximal 1/2 des Wachstums der Kontrollen)
- +: starke Wachstumshemmung (maximal 1/4 des Wachstums der Kontrollen) Das sog. "verminderte Wachstum" (v.W.) umfaßt die Bereiche + bis +++
- 0: absolute Hemmung (a. H.)

### Ergebnisse:

**Tabelle**

| **Prüfung von 4 Thiouroniumformulierungen auf bakteriostatische und fungistatische Wirksamkeit** | | | | | |
|---|---|---|---|---|---|
| Formulierung | minimale Hemmkonzentration in % | | | | KbW₃* |
| | Staphylococcus aureus | Enterococcos faecium | Pseudomonas aeroginosa | Candida albicans | |
| ME 1 | 0,012 | 0,006 | 0,4 | 0,006 | 12,0 |
| ME 2 | 0,025 | 0,012 | 0,4 | 0,006 | 11,4 |
| ME 3 | 0,006 | 0,003 | 0,1 | 0,003 | 13,2 |
| ME 4 | 0,006 | 0,003 | 0,1 | 0,0015 | 13,4 |

| | | | | | |
|---|---|---|---|---|---|
| *Kennzahl der bakteriostatischen Wirksamkeit nach Wolffen et al. | | | | | |

Ein praktischer Versuch in einer automatischen Spülmaschine für chirurgische Instrumente, "Belimed 2000", ergab keine Beeinträchtigung des Programms durch Schaumbildung.

### Beispiel 5: Bakterizide Wirkung einer Mikroemulsionsformulierung mit 5 % Decylthiouroniumsalz

### Prüfkeim: Staphylococcus aureus

| **Zusammensetzung der Mikroemulsionsformulierung:** | |
|---|---|
| Gew.-% | |
| 15,0 % | Decylthiouroniumbromid |
| 5,0 % | Plurafac LF 223⁽³⁾ |
| 5,0 % | Propandiol-1,2 |
| 5,0 % | Butandiol-1,3 |
| 5,0 % | Propanol-2 |
| 8,0 % | Builderlösung (12,5 % Citronensäure/12,5 % Phosphoncarbonsäure pH 3,5) |
| 0,3 % | Benzotriazol |
| 0,3 % | 2-Octyldodecanol-2 als Cotensid |
| | Wasser ad 100 %. |

| | |
|---|---|
| ⁽³⁾ Fettalkylpolyoxybutylenpolyoxyethylen mit einem Trübungspunkt von 33 °C | |

Methodik: Quantitativer Suspensionstest entsprechend der Richtlinie für die Prüfung und Bewertung chemischer Desinfektionsverfahren der Deutschen Gesellschaft für Hygiene und Mikrobiologie. Testdurchführung bei 50 °C.

### Ergebnisse:

Nach einer Einwirkungszeit von 10 min wird eine ausreichende Wirkung durch die 0,5 %ige Lösung erzielt.

**Tabelle:**

| **Bakterizide Wirksamkeit von wäßrigen Formulierungen mit Decylthiouroniumbromid bei 50 °C; Testkeim: Staphylococcus aureus log. Reduktionsfaktoren** | | | | |
|---|---|---|---|---|
| **Konzentration** **(Gew.-%)** | **Einwirkungszeit (min)** | | | |
| | **5** | **10** | **15** | **30** |
| 0,25 | 2,9 | 3,9 | 4,57 | > 5,2 |
| 0,5 | 3,64 | >5,2 | >5,2 | > 5,2 |
| 1 | 3,8 | > 5,2 | > 5,2 | > 5,2 |
| Kontrolle in Wasser standardisierter Härte: 6,2. | | | | |

Ein praktischer Versuch in einer automatischen Spülmaschine für chirurgische Instrumente, "Belimed 2000", ergab keine Beeinträchtigung des Programms durch Schaumbildung.

### Beispiel 6 und 7: Bakterizide Wirkung von Mikroemulsionen bei 50 °C

Untersucht wurden zwei Formulierungen mit folgender Zusammensetzung:
Mikroemulsionsformulierung ME 6:10 % Decylthiouroniumbromid; 5 % Phenoxyethanol: 5 % Propanol-2; 0,15 % Benzotriazol; 3 % Butandiol-1,3; 4 % Fettalkylpolyoxybutylenpolyoxyethylen mit einem Trübungspunkt von 41 °C; 3 % Diethylenglykol; Rest Wasser.
Mikroemulsionsformulierung ME 7:10 % Decylthiouroniumbromid; 5 % Phenoxyethanol; 5 % Propanol-2; 0,15 % Benzotriazol; 3 % Butandiol-1,3; 4 % Fettalkylpolyoxy-butylenpolyoxyethylen mit einem Trübungspunkt von 41 °C; 4 % Propandiol-1,3; Rest Wasser.

| **log. Reduktionsfaktoren: Testkeim: Staphylococcus aureus** | | | | | |
|---|---|---|---|---|---|
| **Mikroemulsion** | **Konzentration** | **Einwirkungszeit in min** | | | |
| | | **5** | **10** | **15** | **30** |
| ME 6 | 0,5 % | 4.17 | 4,94 | > 5,5 | 5,5 |
| | 1 % | > 5,5 | > 5,5 | > 5,5 | 5,5 |
| ME 7 | 0,5 % | 4,94 | > 5,5 | > 5,5 | > 5,5 |
| | 1 % | 5,5 | > 5,5 | > 5,5 | > 5,5 |
| Kontrolle in Wasser standardisierter Härte: 6,51 | | | | | |

Ein praktischer Versuch in einer automatischen Spülmaschine für chirurgische Instrumente, "Belimed 2000", ergab keine Beeinträchtigung des Programms durch Schaumbildung.

### Beispiel 8: Einfluß von Mikroemulsionen mit Thiouroniumsalzen für die maschinelle Instrumentendesinfektion auf die Eiweißfällung

Untersucht wurde eine Formulierung mit folgender Zusammensetzung ME 8: 10 % Decylthiouroniumbromid; 5 % Penoxyethanol; 7,5 % Butanol-2; 0,15 % Benzotriazol; 5 % Ethyllactat; 4 % Fettalkylpolyoxybutylenpolyoxyethylen mit einem Trübungspunkt von 33 °C; 3 % Tetradecanol; 3 % Hexadecan; 0,3 % Linalool; 0,4 % oxethyliertes Fettalkylsulfobetain; Rest Wasser.

| **Tabelle Eiweißfällung aus Ovalbumin** | | | | |
|---|---|---|---|---|
| **Prüfsubstanz** | **Zusatz** | **Ovalbuminverdünnung** | | |
| | µl | 1:16 | 1:32 | 1:64 |
| Decylthiouroniumbromid 0,2 %ige wäßr. Lsg. | 150 | ++ | ++ | ++ |
| Mikroemulsion ME 8 2 %ig | 150 | (+) | (+) | (+) |

| **Tabelle Eiweißfällung aus Humanserum** | | | | | | |
|---|---|---|---|---|---|---|
| **Prüfsubstanz** | **Zusatz** | | **Serumverdünnung** | | | |
| | µl | 1:2 | 1:4 | 1:8 | 1:16 | 1:32 |
| Decylthiouroniumbromid 0,2 %ige wäßr. Lsg. | | | | | | |
| | 150 | + | (+) | ++ | ++ | ++ |
| | 200 | ++ | ++ | ++ | ++ | ++ |
| | 300 | ++ | ++ | ++ | ++ | ++ |
| | 400 | ++ | ++ | ++ | ++ | ++ |
| Mikroemulsion ME 8 2 %ig | | | | | | |
| | 150 | 0 | (+) | 0 | 0 | 0 |
| | 200 | 0 | + | (+) | (+) | (+) |
| | 300 | (+) | + | + | + | + |
| | 400 | (+) | + | + | + | + |

### Beispiel 9 und 10: Einfluß auf Hepatitis-B-Oberflächenantigen (HBsAg)-positive Seren

Untersucht wurden zwei Formulierungen mit folgender Zusammensetzung ME 9 und ME 10:
ME 9 bestehend aus: 10 % Decylthiouroniumbromid; 6 % Phenoxyethanol; 8 % Butanol-2; 5 % Ethyllactat; 1 % Fettalkylpolyoxybutylenpolyoxyethylen mit einem Trübungspunkt von 41 °C; 0,3 % Citronellol; 3 % Tetradecanol; 7 % Isopropylmyristat; 0,3 % Benztriazol: 0,4 % Fettalkyldimethylammoniopropansulfonat; Rest Wasser.
ME 10 mit der Zusammensetzung:10 % Decylthiouroniumbromid; 6 % Phenoxyethanol; 8 % Butanol-2; 5 % Ethyllactat; 1 % Fettalkylpolyoxybutylenpolyoxyethylen mit einem Trübungspunkt von 33 °C; 0,3 % Citronellol; 3 % Tetradecanol; 7 % Isopropylmyristat; 0,3 % Benztnazol; 0,4 % Fettalkyldimethylammoniopropansulfonat; Rest Wasser.

Methodik: HBsAg-haltiges Serum wurde im Verhältnis 1:19 mit 1, 1,5 und 2 %igen Lösungen des Desinfekionsmittels versetzt.
Bei Temperaturen von 45-60°C wirkte das Desinfektionsmittel 5 min ein. Danach erfolgte eine Inaktivierung durch Verdünnung im Verhältnis 1:100 mit eiskaltem MEIA-Puffer (Mikropartikel-lmmuno-Assay-Puffer). Der Nachweis des HBsAg erfolgte mit dem Mikropartikelenzymimmunoassay mit dem IMx (Abbott Dianostica GmbH, Wiesbaden).

### Ergebnis:

Das HBsAg wird durch die Mikroemulsion inaktiviert.

| **Tabelle Inaktivierung von HBsAg** | | | | |
|---|---|---|---|---|
| Temparatur-Nachweis von HBsAg | | | | |
| | **°C** | **1%** | **1,5%** | **2%** |
| ME 10 | 45 | - | - | - |
| Kontrolle | 45 | + | + | + |
| ME 9 | 50 | - | - | - |
| ME 10 | 50 | - | - | - |
| Kontrolle | 50 | + | + | + |
| ME 10 | 55 | - | - | - |
| Kontrolle | 55 | + | + | + |
| ME 10 | 60 | - | - | - |
| Kontrolle | 60 | + | + | + |

### Beispiel 11: Mikroemulsionen von Thiouroniumsalzen mit sporicider Wirkung

Untersucht wurde eine Formulierung ME 11 mit folgender Zusammensetzung: 10 % Dodecylthiouroniumbromid; 5 % Phenoxyethanol; 5 % Butandiol-1,3; 5 % Butandiol-2; 3 % Fettalkylpolyoxybutylenpolyoxyethylen mit einem Trübungspunkt von 39 °C; 2 % Tetradecanol; Rest Wasser.

Methodik: Kommerzielle Teststreifen mit 10⁶ Sporen von Bacillus subtilis variation niger ATCC 9372 (BAG-Bio Strips, Biologische Analysensystem GmbH Lich) wurden für 3 Tage in eine Lösung der Prüfsubstanz eingelegt. Danach wurden die Teststreifen in eine Nährlösung überführt und 7 Tage bebrütet.

### Ergebnis:

Bei einer Konzentration > 0,05 % werden die Sporen vollständig abgetötet.

**Tabelle:**

| **Ergebnisse der Sporicidieprüfung der Mikroemulionsformulierung ME 11** | | |
|---|---|---|
| Konzentration | n | davon ohne Wachstum |
| 1,0 | 3 | 3 |
| 0,5 | 3 | 3 |
| 0,1 | 18 | 18 |
| 0,05 | 21 | 21 |
| 0,025 | 3 | 2 |

### Beispiel 12: Kombination von Decylthiouroniumsalz mit Benzoesäureestern

Es wurde eine Mikroemulsion ME 12, die 10 Gew.-% Decylthiouroniumbromid, 5 Gew.-% Propyl-4-hydroxybenzoat, 5 Gew.-% Methyl-4-hydroxybenzoat sowie 5 Gew.-% Ethyllactat. 8 Gew.-% Butanol-2, 6 Gew.-% Isopropylmyristat sowie 2 Gew.-% Fettalkylpolyoxyethylen in Wasser enthielt, im Vergleich zu den wäßrigen Lösungen der reinen Stoffe (Decylthiouroniumbromid und p-Hydroxybenzoesäureester) untersucht. Die fungistatische Wirksamkeit gegenüber Candida albicans wurde im Reihenverdünnungstest bestimmt (s. Beispiel 1).

| **Prüfsubstanz** | **Minimale Hemmkonzentration** |
|---|---|
| Gemisch aus Methyl- und Propyl-4-hydroxybenzoat | 0,098 % |
| Decylisothioroniumbromid | 0,098 % |
| Mikroemulsion ME 12 | 0,012 % |

Nachweis des Synergismus: q_{A}+_{B} =0,0012/0,098 + 0,0012/0,098 = 0,024

### Beispiel 13: Verminderung der eiweißfällenden Wirkung bei Kombination mit Thiouroniumsalzen

Es wurde die Mikroemulsion ME 12 aus Beispiel 12 im Vergleich zu einer 0,2 Gew.-%igen wäßrigen Decylthiouroniumbromidlösung auf Eiweißfällung untersucht. Die Prüfung auf Eiweißfällung erfolgte mit verdünntem humanen Serum sowie mit Ovalbuminlösungen. Die Reaktion wurde 5 und 60 min nach Mischung von je 150 µl der Lösung der Prüfsubstanzen mit 1 ml der verdünnten Eiweißlösungen visuell nach folgendem Schema abgeschätzt:
- 0: keine Veränderung
- (+): sehr geringe Trübung
- +: leichte Trübung
- ++: starke Trübung
- +++: Ausfällung

### Ergebnisse:

Im Gegensatz zu dem Thiouroniumsalz als Einzelsubstanz wirkt die Kombination mit Methyl- bzw. Propyl-4-hydroxybenzoat oder ein Gemisch beider Ester nicht eiweißfällend.

| **Tabelle Eiweißfällung aus Humanserum** | | | | | | | |
|---|---|---|---|---|---|---|---|
| **Prüfsubstanz** | **Zusatz** | **Serumverdünnung** | | | | | |
| | µl | 1:2 | 1:4 | 1:8 | 1:16 | 1:32 | 1:64 |
| Decylthiouroniumbromid, 0,2 %ig | 150 | + | (+) | ++ | ++ | ++ | (+) |
| | 160 | ++ | + | ++ | ++ | ++ | + |
| | 170 | ++ | + | ++ | ++ | ++ | + |
| | 180 | ++ | + | ++ | ++ | ++ | + |
| Mikroemulsion ME 12;1 %ig | 150 | 0 | 0 | 0 | 0 | 0 | 0 |
| | 160 | 0 | (+) | 0 | 0 | 0 | 0 |
| | 170 | 0 | (+) | 0 | 0 | 0 | 0 |
| | 180 | 0 | (+) | 0 | 0 | 0 | 0 |

| **Tabelle Eiweißfällung aus Ovalbumin** | | | | | | | |
|---|---|---|---|---|---|---|---|
| **Prüfsubstanz** | **Zusatz** | | **Ovalbuminverdünnung** | | | | |
| | µl | 1:2 | 1:4 | 1:8 | 1:16 | 1:32 | 1:64 |
| Decylthiouroniumbromid, 0,2 %ig | 150 | +++ | +++ | +++ | ++ | ++ | ++ |
| Mikroemulsion ME 12; 1 %ig | 150 | +++ | +++ | 0 | 0 | 0 | 0 |

### Beispiel 14 und 15: Kombinationen mit quarternären Ammoniumverbindungen

Es wurden die Mikroemulsionen ME 13 mit 10 % Decylthiouroniumbromid, 10 % Benzalkoniumchlorid, 0,5% Fettalkylpolyoxybutylenpolyoxyethylen mit einem Trübungspunkt von 45 °C und Wasser sowie eine Lösung L 14 bestehend aus 10 % Decylthiouroniumbromid, 10 % Benzalkoniumchlorid, ohne nichtionische Tenside in Wasser im Vergleich zu den Ausgangsstoffen untersucht. Die Prüfung auf fungistatische Wirksamkeit gegen Candida albicans erfolgte nach der Methodik von Beispiel 1.

### Ergebnisse:

Gegenüber Candida albicans wird ein Synergismus beobachtet.

Tabelle fungistatische Wirksamkeit von Kombinationen von Thiouroniumsalzen und quarternären Ammoniumverbindungen

| **Prüfsubstanz** | **Hemmkonzentration** |
|---|---|
| Benzalkoniumchlorid | 0,003 % |
| Decylthiouroniumbromid | 0,098 % |
| ME 13 | 0,012% |
| L 14 | 0,012 % |

Nachweis des Synergismus q_{A}+_{B} = 0,0012/0,003 + 0,0012/0,098 = 0,41.

### Beispiel 16: Verminderung der Eiweißfällung bei Kombination von Thiouroniumsalzen und quarternären Ammoniumverbindungen

Formulierungen siehe Beispiele 14 und 15.

Die Prüfung auf Eiweißfällung erfolgte mit verdünntem humanen Serum.
Methodik: s. Beispiel 13
Ergebnisse: Die Eiweißfällung wird deutlich vermindert.

**Tabelle:**

| **Eiweißfällung von Serum** | | | | | | |
|---|---|---|---|---|---|---|
| **Prüfsubstanz** | **Serumverdünnung** | | | | | |
| | 1:2 | 1:4 | 1:8 | 1: 16 | 1 : 32 | 1 : 64 |
| Benzalkoniumchlorid 0,2 %ig | ++ | + | + | ++ | ++ | ++ |
| Decylthiouriniumbromid 0,2 %ig | + | (+) | ++ | ++ | ++ | (+) |

## Patentansprüche

1. O/W-Mikroemulsionen **gekennzeichnet durch** einen Gehalt an mindestens einem Alkylthiouronium- und/oder α, ω-Alkylendithiouroniumsalz.

2. O/W-Mikroemulsionen nach Anspruch 1, **dadurch gekennzeichnet, daß** zusätzlich weitere mikrobizide Wirkstoffe enthalten sind.

3. O/W-Mikroemulsionen nach Anspruch 1, **dadurch gekennzeichnet, daß** zusätzlich Tenside und/oder Hilfs-, Zusatz- und/oder Wirkstoffe enthalten sind.

4. O/W-Mikroemulsionen nach einem der vorhergehenden Ansprüche, **dadurch gekennzeichnet, daß** der Gehalt an einem oder mehreren Alkylthiouronium- und/oder α, ω-Alkylendithiouroniumsalzen 0,05 bis 30 Gew.-%, bezogen auf das Gesamtgewicht der Mikroemulsion, beträgt.

5. O/W-Mikroemulsionen nach einem der vorhergehenden Ansprüche, **dadurch gekennzeichnet, daß** der oder die Emulgatoren aus der Gruppe der nichtionischen Emulgatoren, insbesondere der oxethylierten geradkettigen Alkohole gewählt werden.

6. O/W-Mikroemulsionen nach einem der vorhergehenden Ansprüche, **dadurch gekennzeichnet, daß** zusätzlich Substanzen mit sehr geringer Schaumneigung bzw. schaumdämpfender Wirkung enthalten sind, insbesondere Fettalkylpolyoxyalkylene.

7. O/W-Mikroemulsionen nach einem der vorhergehenden Ansprüche, **dadurch gekennzeichnet, daß** es sich um Konzentrate handelt, die vor der Verwendung mit Wasser zu einer Gebrauchslösung verdünnt werden.

8. Verwendung von O/W-Mikroemulsionen nach Anspruch 1 als Desinfektionsmittel für medizinisch-chirurgische Instrumente und Flächen.

9. Verwendung von O/W-Mikroemulsionen nach Anspruch 1 als Desinfektionsmittel für medizinisch-chirurgische Instrumente in Wasch- und/oder Desinfektionsautomaten.

## Claims

1. O/W microemulsions **characterized in that** they contain at least one alkylthiouronium and/or α,ω-alkylenedithiouronium salt.

2. O/W microemulsions according to Claim 1, **characterized in that** further microbicidal active compounds are additionally contained.

3. O/W microemulsions according to Claim 1, **characterized in that** surfactants and/or excipients, additives and/or active compounds are additionally contained.

4. O/W microemulsions according to one of the preceding claims, **characterized in that** the content of one or more alkylthiouronium and/or α,ω-alkylene-dithiouronium salts is 0.05 to 30% by weight, based on the total weight of the microemulsion.

5. O/W microemulsions according to one of the preceding claims, **characterized in that** the emulsifier or the emulsifiers are chosen from the group consisting of the non-ionic emulsifiers, in particular of the ethoxylated straight-chain alcohols.

6. O/W microemulsions according to one of the preceding claims, **characterized in that** substances having very low proneness to foaming or foam-damping action are additionally contained, in particular fatty alkyl polyoxyalkylenes.

7. O/W microemulsions according to one of the preceding claims, **characterized in that** they are concentrates which are diluted with water to give a use solution before use.

8. Use of O/W microemulsions according to Claim 1 as disinfectants for medical/surgical instruments and surfaces.

9. Use of O/W microemulsions according to Claim 1 as disinfectants for medical/surgical instruments in washing and/or disinfection machines.

## Revendications

1. Microémulsions H/E, **caractérisées par** une teneur en au moins un sel d'alkylthio-uronium et/ou d'α,ω-alkylènedithio-uronium.

2. Microémulsions H/E selon la revendication 1, **caractérisées en ce que** d'autres substances actives microbicides sont en outre contenues.

3. Microémulsions H/E selon la revendication 1, **caractérisées en ce que** des tensioactifs et/ou des adjuvants, additifs et/ou principes actifs sont en outre contenus.

4. Microémulsions H/E selon l'une quelconque des revendications précédentes, **caractérisées en ce que** la teneur en un ou plusieurs sels d'alkylthio-uronium et/ou d'α,ω-alkylènedithio-uronium va de 0,05 à 30 % en poids, par rapport au poids total de la microémulsion.

5. Microémulsions H/E selon l'une quelconque des revendications précédentes, **caractérisées en ce que** le ou les émulsifiants sont choisis dans le groupe des émulsifiants non ioniques, en particulier des alcools oxyéthylés à chaîne droite.

6. Microémulsions H/E selon l'une quelconque des revendications précédentes, **caractérisées en ce que** des substances à très faible tendance à la formation de mousse ou à effet antimousse sont en outre contenues, en particulier des (alkyl gras)polyoxyalkylènes.

7. Microémulsions H/E selon l'une quelconque des revendications précédentes, **caractérisées en ce qu'**il s'agit de concentrés qui sont dilués avant l'emploi, avec de l'eau, en une solution prête à l'emploi.

8. Utilisation de microémulsions H/E selon la revendication 1, en tant que désinfectant pour instruments médicaux-chirurgicaux et surfaces.

9. Utilisation de microémulsions H/E selon la revendication 1, en tant que désinfectant pour instruments médicaux-chirurgicaux et/ou machines automatiques de désinfection.
